(19) Europäisches Patentamt
European Patent Office
Office européen des brevets

(11) Veröffentlichungsnummer : **0 154 977 B1**

(12) **EUROPÄISCHE PATENTSCHRIFT**

(45) Veröffentlichungstag der Patentschrift : **17.05.89**

(51) Int. Cl.[4] : **C 07 F 9/10**, C 07 F 9/65, A 61 K 47/00

(21) Anmeldenummer : **85102830.8**

(22) Anmeldetag : **12.03.85**

(54) **Glycerinäther-phosphatide, ihre Herstellung und Verwendung.**

(30) Priorität : **15.03.84 CH 1287/84**
**04.02.85 CH 491/85**

(43) Veröffentlichungstag der Anmeldung : **18.09.85 Patentblatt 85/38**

(45) Bekanntmachung des Hinweises auf die Patenterteilung : **17.05.89 Patentblatt 89/20**

(84) Benannte Vertragsstaaten : **AT BE CH DE FR GB IT LI LU NL SE**

(56) Entgegenhaltungen :
**EP-A- 0 056 781**
**GB-A- 2 020 663**
**JP-A-59 163 389**
**CHEMICAL ABSTRACTS, Band 92, Nr. 23, 9. Juni 1980, Seite 635, Nr. 197869u, Columbus, Ohio, US; V.A. VAVER u.a.: "Synthesis of 2,3-di-O-phytanyl-sn-glycero-1-phosphorylcholine", & BIOORG. KHIM. 1980, 6(1), 146-8**
**Chemical Abstracts, vol. 102 (1985), abstract no. 149526h (US)**
**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**
**CH-4002 Basel (CH)**

(72) Erfinder : **Breuninger, Manfred, Dr.**
**Habsburgerstrasse 71**
**D-7800 Freiburg/Brsg. (DE)**
Erfinder : **Schmidt, Dieter, Dr.**
**Redingstrasse 20**
**CH-4053 Basel (CH)**

(74) Vertreter : **Lederer, Franz, Dr. et al**
**Van der Werth, Lederer & Riederer Patentanwälte**
**Lucile-Grahn-Strasse 22**
**D-8000 München 80 (DE)**

**EP 0 154 977 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Glycerinätherphosphatide der allgemeinen Formel

$$\begin{array}{l} CH_2\text{-}O\text{-}R^1 \\ | \\ CH\ \text{-}O\text{-}R^2 \\ | \\ CH_2\text{-}O\text{-}R^3 \end{array} \qquad (I)$$

worin zwei der Reste $R^1$, $R^2$ und $R^3$ $C_{10\text{-}30}$-Alkylreste mit mindestens 8 C-Atomen in gerader Kette darstellen, wobei mindestens einer dieser Reste durch mindestens 2 $C_{1\text{-}3}$-Alkylreste substituiert ist und die Summe der C-Atome in beiden Resten grösser als 20 ist ; und der dritte Rest ein Rest $-P(O)(O^-)OR^4$ ist, worin $R^4$ einen durch eine quaternäre Ammoniumgruppe substituierten Butyl- oder $C_{5\text{-}7}$-Cycloalkylrest oder einen ein di-($C_{1\text{-}6}$-Alkyl)-substituiertes Stickstoffatom enthaltenden $C_{5\text{-}7}$-Cycloalkylrest darstellt.

Die Alkylreste $R^1$, $R^2$ und $R^3$ sind vorzugsweise Terpenkohlenwasserstoffreste. Beispiele solcher Terpenkohlenwasserstoffreste sind Reste der Formel

$$\left[-CH_2-CH_2-\overset{CH_3}{\overset{|}{CH}}-CH_2-\right]_n CH_2-CH_2-\overset{CH_3}{\overset{|}{CH}}-CH_2-CH_2-CH_2-\overset{CH_3}{\overset{|}{CH}}-CH_3$$

worin n eine ganze Zahl von 0-4 ist, wie Tetrahydrogeranyl, Hexahydrofarnesyl und insbesondere Dihydrophytyl.

Beispiele für Reste $R^4$ Trimethylammoniobutyl ; Tri-($C_{1\text{-}6}$-alkyl)-ammonio-$C_{5\text{-}7}$-cycloalkyl, wie Trimethylammoniocyclohexyl ; und N,N-Di-$C_{1\text{-}6}$-alkyl-$C_{4\text{-}6}$-azacycloalkyl, wie N,N-Dimethylpiperidyl. Die im Rest $R^4$ enthaltene quaternäre Ammoniumgruppe kann auch durch ein Stickstoffatom eines 5-7-gliedrigen heterocyclischen Rings gebildet werden. Beispiele solcher Reste $R^4$ sind (N-$C_{1\text{-}6}$-Alkyl-piperidyl)-$C_{1\text{-}6}$-alkyl wie (N-Methylpiperidinyl)-äthyl. Der Ausdruck $C_{1\text{-}6}$ bezeichnet Reste mit 1-6 C-Atomen, wie Methyl, Aethyl, Propyl, Butyl.

Eine bevorzugte Gruppe von Verbindungen der Formel I sind diejenigen in denen $R^1$ und $R^2$ $C_{10\text{-}30}$-Alkylreste mit mindestens 8 C-Atomen in gerader Kette darstellen, die jeweils durch mindestens 2 $C_{1\text{-}3}$-Alkylreste substituiert sind.

Bevorzugte Reste $R^4$ sind 4-(Trimethylammonio)butyl, 4-(Trimethylammonio)cyclohexyl und N,N-Dimethyl-4-piperidyl.

Die Verbindungen der Formel I können erfindungsgemäss dadurch hergestellt werden, dass man einen Glycerinäther der allgemeinen Formel

$$\begin{array}{l} CH_2\text{-}O\text{-}R^{11} \\ | \\ CH\ \text{-}O\text{-}R^{21} \\ | \\ CH_2\text{-}O\text{-}R^{31} \end{array} \qquad (II)$$

wobei einer der Reste $R^{11}$, $R^{21}$ und $R^{31}$ Wasserstoff und die verbleibenden Reste einen wie oben definierten $C_{10\text{-}30}$-Alkylrest darstellen,

a) mit Phosphoroxychlorid in Gegenwart einer Base und danach mit einem Alkohol $R^4OH$, worin $R^4$ die oben angegebene Bedeutung hat, umsetzt, oder

b) in Gegenwart einer Base mit einer Verbindung der Formel

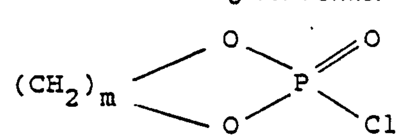

(III)

worin m 2 oder 3 ist, und danach mit einem Tri-$C_{1\text{-}6}$-alkylamin umsetzt.

Als Basen kommen insbesondere organische Basen, z. B. tertiäre Amine wie Triäthylamin, Pyridin

oder Collidin in Betracht. Die Reaktion wird zweckmässig in einem inerten organischen Lösungsmittel, z. B. einem Kohlenwasserstoff wie Benzol oder Toluol oder einem chlorierten Kohlenwasserstoff, wie Chloroform, vorgenommen. Zweckmässig wird die Reaktion bei niedrigen Temperaturen, z. B. bei 0 °C bis Raumtemperatur, durchgeführt, wobei bei der Umsetzung einer Verbindung II mit Phosphoroxichlorid letzteres zweckmässig bei tiefen Temperaturen, z. B. bei — 78 °C bis — 10 °C zugesetzt wird.

Die Verbindungen der Formel I können zur Herstellung kolloidaler Lösungssysteme, wie Liposomen- und Mischmicellösungen z. B. zur Solubilisierung fettlöslicher Arzneimittel in wässrigen Systemen in ähnlicher Weise wie natürliche Lecithine verwendet werden.

Aus Chemical Abstracts 92 : 197869u ist ein 2,3-Di-O-phytanyl-sn-glycero-1-phosphorylcholin als Modellsubstanz für Untersuchungen der Funktion von Bakteriorhodopsin bekannt. Weiterhin ist es aus der EP-A-56 781 bekannt, Liposomenlösungen von Arzneimitteln mit Phosphoglyceriddiäthern herzustellen.

Gegenüber natürlichen Lecithinen weisen die neuen Verbindungen der Formel I z. B. den Vorteil einer grösseren chemischen Stabilität auf. Weiterhin können sie im Gegensatz zu natürlichen Lecithinen in chemisch einheitlicher Form bereitgestellt werden.

Zur Herstellung von Mischmicellen können die neuen Verbindungen insbesondere mit Gallensäuren und deren Salzen, z. B. Cholsäure, Glycocholsäure, Taurocholsäure, Deoxycholsäure, Glycodeoxycholsäure, Chenodeoxycholsäure kombiniert werden.

Die auf der Basis der erfindungsgemässen Verbindungen herstellbaren Liposomen- und Mischmicell-Lösungen können zur Solubilisierung von in Wasser schwer löslichen oder unlöslichen Pharmaka, z. B. Benzodiazepinen, wie Diazepam, Nitrazepam, Flunitrazepam, Medazepam und Bromazepam, oder fettlöslichen Vitaminen wie Vitamin A, D, E und K Verwendung finden. Die auf der Basis der erfindungsgemässen Verbindungen hergestellten Liposomenlösungen können zur Verbesserung der Stabilität Zucker, z. B. Mono- oder Disaccharide wie Glucose, Fructose, oder Saccharose ; oder zuckerähnliche Polyalkohole wie Sorbit oder Xylit enthalten.

Die Verbindungen der Formel I liegen als innere Salz vor. Sie enthalten chirale Zentren und können daher in verschiedenen enantiomeren Formen vorliegen, die ebenfalls Gegenstand der Erfindung sind.

Die Verbindungen der Formel II können ausgehend von Glycerinderivaten, in denen eine oder zwei Hydroxygruppen in geschützter Form vorliegen, in in den Beispielen näher ausgeführten Weise hergestellt werden.

Die Erfindung wird durch die nachfolgenden Beispiele weiter erläutert.

Beispiel A (Referenzbeispiel)

8,4 mmol Triäthylamin (über KOH destilliert) wurden in 5 ml Chloroform (über Aluminiumoxid getrocknet) bei — 78 °C unter Rühren mit 2,2 mmol frisch destilliertem Phosphoroxichlorid versetzt. Das Kühlbad wurde durch ein Eisbad ersetzt und danach wurden 2,09 mmol (RS)-2,3-bis-[[(3RS,7R,11R)-3,7,11,15-Tetramethylhexadecyl]oxi]propanol in 10 ml trockenem Chloroform gelöst, zugetropft. Danach wurde noch 1 Stunde ohne weitere Kühlung gerührt. Nach erneuter Kühlung auf 0 °C wurden 3,2 mmol Cholintosylat in ca. 20 ml trockenem Pyridin im Verlauf von etwa 30 Minuten unter Rühren zugegeben. Danach wurde das Reaktionsgemisch noch einige Stunden bei Raumtemperatur gerührt und dann über Nacht stehengelassen. Zur Aufarbeitung wurde das Chloroform im Wasserstrahlvakuum bei 30 °C entfernt, der Rückstand mit 20 mmol Kaliumhydrogencarbonat und 5 ml Wasser versetzt und das Gemisch im Wasserstrahlvakuum zur Trockne eingedampft. Der Rückstand wurde in Dichlormethan aufgenommen, mit Toluol versetzt, filtriert und im Wasserstrahlvakuum eingedampft. Der Rückstand wurde in ca. 30 ml Tetrahydrofuran/Wasser (95 : 5) mehrmals langsam über eine Säule mit 25 g Ionenaustauscher Amberlit MB-83 gegeben. Danach wurde der Ionenaustauscher mit dem gleichen Lösungsmittel gut ausgewaschen. Filtrat und Waschlösung wurden im Wasserstrahlvakuum eingedampft, Wasserreste wurden durch Abdampfen mit Aethanol entfernt. Das so erhaltene Rohprodukt wurde an 100 g Kieselgel chromatographiert. Mit Chloroform/Methanol (7 : 3) wurden gefärbte Verunreinigungen, danach mit Chloroform/Methanol/Wasser (60 : 35 : 5) das Reaktionsprodukt, O-[[(RS)-2,3-Bis[[3RS,7R,11R)-3,7,11,15-Tetramethylhexadecyl]oxi]propyl]hydroxiphosphinyl]cholinhydroxid (inneres Salz) erhalten.

NMR : 0,75-0,95 (Multiplett, $CH_3$) 1,0-1,8 (breites Multiplett, $CH_2$ und CH) 3,39 (breites Siglett, $CH_3$—N) 3,3-3,7, 3,7-4,1 und 4,1-4,5 (3 breite Multipletts, $CH_2$—N, $CH_2$—O und CH—O).

Elementaranalyse für $C_{48}H_{100}NO_6P$ :

Berechnet : C 70,45 H 12,32 N 1,71
Gefunden : C 70,44 H 12,63 N 1,82 (3,04 % Wasser).

Die Ausgangsstoffe können wie folgt hergestellt werden :

a) 50 mmol Dimethylaminoäthanol wurden in 25 ml trockenem Aether mit einer Lösung von 50 mmol p-Toluolsulfonsäuremethylester in 50 ml trockenem Aether versetzt. Das Reaktionsgemisch wurde über Nacht bei Raumtemperatur unter Feuchtigkeitsausschluss stehengelassen. Das Cholintosylat wurde unter Feuchtigkeitsausschluss abgesaugt, mit trockenem Aether gewaschen und trocken aufbewahrt.

b) Eine Lösung von 1,0 mol Dihydrophytol in 1 500 ml trockenem Dichlormethan wurde mit 300 ml trockenem Pyridin und 1,1 mol Toluolsulfochlorid unter Rühren versetzt. Die Lösung wurde über Nacht bei Raumtemperatur stehengelassen, danach wurde das Lösungsmittel bei 30° (Badtemperatur) weitgehend abdestilliert und der Rückstand in Aether aufgenommen. Die Lösung wurde eingeengt, mit 200 ml Wasser, 100 g Natriumhydrogencarbonat und 100 ml Pyridin versetzt und 1 Stunde gerührt. Danach wurde die Lösung bei 60 °C zur Trockne eingedampft, der Rückstand mit 1 000 ml Toluol versetzt und das Toluol unter vermindertem Druck abdestilliert. Der Rückstand wurde in 500 ml Toluol aufgenommen, die Lösung filtriert und das Lösungsmittel entfernt, wobei das (3RS,7R,11R)-3,7,11,15-Tetramethylhexadecyl-p-toluolsulfonat erhalten wurde.

c) 0,8 g Natriumhydrid (80 %ig) wurden mit 2 mal 10 ml Pentan gewaschen und mit 80 ml Dimethylformamid und 1,8 g 1-O-Benzylglycerin in 10 ml Dimethylformamid versetzt. Das Reaktionsgemisch wurde 1 Stunde bei 50-60 °C unter Feuchtigkeitsausschluss gerührt, mit 22 mmol (3RS,7R,11R)-3,7,11,15-tetramethylhexadecyl-p-toluolsulfonat in 20 ml Dimethylformamid versetzt und 1-4 Stunden bei 50-60 °C gerührt. Danach wurden 5 ml Wasser und 20 ml Aethanol zugegeben, die Lösungsmittel wurden bei 60 °C im Wasserstrahlvakuum abdestilliert und der Rückstand in Aether aufgenommen. Nach Aufarbeitung der ätherischen Lösung wurde das (RS)-1-(Benzyloxy)-2,3-bis[[(3RS,7R,11R)-3,7,11,15-tetramethylhexadecyl]oxi]propan als farbloses Oel erhalten.

d) 4 g (5,4 mmol) des in Abschnitt c) erhaltenen Benzyläthers wurden in einem Gemisch von 25 ml Tetrahydrofuran und 25 ml Aethanol über 0,2 g Pd-C (5 %) hydriert und gaben nach Chromatographie das (RS)-2,3-Bis[[(3RS,7R,11R)-3,7,11,15-tetramethylhexadecyl]oxi]propanol.

## Beispiel 1

In Analogie zu Beispiel A wurden aus 0,9 g (2,4 mmol) (RS)-2,3-Bis[[3RS,7RS)-3,7,11-trimethyldodecyl]oxi]propanol und 1,2 g (3,95 mmol) (4-Hydroxibutyl)-trimethylammonium-p-toluolsulfonat 0,3 g (17,6 %) [4-[[[(RS)-2,3-Bis[[(3RS,7RS)-3,7,11-trimethyldodecyl]oxi]propoxy]hydroxiphosphinyl]oxi]butyl]trimethylammoniumhydroxid (inneres Salz) erhalten.

NMR : 0,78-0,90 (Multiplett, $CH_3$), 1,05-2,13 (breites Multiplett, $CH_2$ und CH), 3,14 (Singlett, $CH_3$—N), 3,40-4,13 (breites Multiplett, $CH_2$—N, $CH_2$—O und CH—O).

Elementaranalyse für $C_{40}H_{84}NO_6P$ :

Berechnet : C 68,04 H 11,99 N 1,98
Gefunden : C 67,70 H 12,26 N 2,07 (1,31 % Wasser).

## Beispiel 2

In Analogie zu Beispiel A, wurden aus 1 g (1,53 mmol) (RS)-2,3-Bis-[[3RS,7R,11R)-3,7,11,15-Tetramethylhexadecyl]oxi]propanol und 0,7 g (2,3 mmol) (4-Hydroxibutyl)-tri-methylammonium-p-toluolsulfonat 0,78 g (60,2 %) [4-[[[(RS)-2,3-Bis[[(3RS,7R,11R)-3,7,11,15-tetramethylhexadecyl]oxi]propoxi]hydroxiphosphinyl]oxi]butyl]trimethylammoniumhydroxid (inneres Salz) erhalten.

NMR : 0,78-0,95 (Multiplett, $CH_3$), 1,05-2,11 (breites Multiplett, $CH_2$ und CH), 3,11 (Singlett, $CH_3$—N), 3,33-4,02 (breites Multiplett, $CH_2$—N, $CH_2$—O und CH—O).

Elementaranalyse für $C_{50}H_{104}NO_6P$ :

Berechnet : C 70,96 H 12,39 N 1,65
Gefunden : C 71,20 H 12,34 N 1,84 (0,98 % Wasser).

Das (4-Hydroxybutyl)-trimethyl-ammonium-p-toluolsulfonat wurde in Analogie zu Beispiel 1, Abschnitt a) aus 4-Dimethylaminobutanol hergestellt.

## Beispiel 3

In Analogie zu Beispiel A wurden aus 12 g (18,4 mmol) (RS)-2,3-Bis[[(3RS,7RS,11RS)-3,7,11,15-Tetramethylhexadecyl]oxi]propanol und 10,4 g (34,3 mmol) (4-Hydroxibutyl)-trimethylammonium-p-toluolsulfonat 8 g (51,4 %) [4-[[[(RS)-2,3-Bis[[(3RS,7RS,11RS)-3,7,11,15-Tetramethylhexadecyl]oxi]propoxi] hydroxiphosphinyl]oxi]butyl]trimethylammoniumhydroxid (inneres Salz) erhalten.

NMR : praktisch identisch mit dem Spektrum der Verbindung von Beispiel 10.

Elementaranalyse für $C_{50}H_{104}NO_6P$ :

Berechnet : C 70,96 H 12,39
Gefunden : C 70,52 H 12,37 (0,25 % Wasser).

## Beispiel 4

In Analogie zu Beispiel A wurden aus 1 g (1,71 mmol) (RS-)1-O-[(3RS,7R,11R)-3,7,11,15-Tetramethyl-

hexadecyl]-2-O-[(3RS,7RS)-3,7,11-trimethyldodecyl]glycerin und 0,78 g (2,6 mmol) (4-Hydroxibutyl)-trimethylammonium-p-toluolsulfonat 0,83 g [4-[[Hydroxi-[(RS)-3-[[(3RS,7R,11R)-3,7,11,15-tetramethylhexadecyl]oxi]-2-[[(3RS,7RS)-3,7,11-trimethyldodecyl]oxi]propoxi]phosphinyl]oxi]butyl]trimethylammoniumhydroxid (inneres Salz) erhalten.

NMR : 0,84-0,92 (Multiplett, $CH_3$), 1,05-2,10 (breites Multiplett, $CH_2$ und CH), 3,12 (Singlett, $CH_3$—N), 3,30-4,05 (breites Multiplett, $CH_2$—N, $CH_2$—O und CH—O).

Elementaranalyse für $C_{45}H_{94}NO_6P$ :

| | | | |
|---|---|---|---|
| Berechnet : | C 69,63 | H 12,21 | N 1,80 |
| Gefunden : | C 69,71 | H 12,48 | N 2,17 (1,23 % Wasser). |

## Beispiel 5

In Analogie zu Beispiel A wurden aus 1,2 g (2,34 mmol) (RS)-2-O-[(RS)-3,7-Dimethyloctyl]-1-O-[(3RS,7R,11R)-3,7,11,15-tetramethylhexadecyl]glycerin und 1,06 g (3,49 mmol) (4-Hydroxibutyl)-trimethylammonium-p-toluolsulfonat 0,7 g (42,3 %) [4-[[Hydroxi-[(RS)-3-[[(3RS,7R,11R)-3,7,11,15-tetramethylhexadecyl]oxi]-2-[[(RS)-3,7-dimethyloctyl]oxi]propoxi]phosphinyl]oxi]butyl]trimethylammoniumhydroxid (inneres Salz) erhalten.

NMR : 0,80-0,97 (Multiplett, $CH_3$), 1,05-2,30 (breites Multiplett, $CH_2$ und CH), 3,18 (Singlett, $CH_3$—N), 3,38-4,15 (breites Multiplett, $CH_2$—N, $CH_2$—O und CH—O).

Elementaranalyse für $C_{40}H_{84}NO_6P$ :

| | | | |
|---|---|---|---|
| Berechnet : | C 68,04 | H 11,99 | N 1,98 |
| Gefunden : | C 68,37 | H 11,77 | N 1,99 (2,46 % Wasser). |

## Beispiel 6

In Analogie zu Beispiel A wurden aus 0,91 g (1,56 mmol) (RS)-2-O-[(3RS,7R,11R)-3,7,11,15-Tetramethylhexadecyl]-1-O-[(3RS,7RS)-3,7,11-trimethyldodecyl]glycerin und 0,71 g (2,34 mmol) (4-Hydroxibutyl)-trimethylammonium-p-toluolsulfonat 0,85 g [4-[[Hydroxi-[(RS)-2-[[(3RS,7R,11R)-3,7,11,15-tetramethylhexadecyl]oxi]-3-[[(3RS,7RS)-3,7,11-trimethyldodecyl]oxi]propoxi]phosphinyl]oxi]butyl]trimethylammoniumhydroxid (inneres Salz) erhalten.

NMR : 0,80-0,97 (Multiplett, $CH_3$), 1,05-2,44 (breites Multiplett, $CH_2$ und CH), 3,14 (Singlett, $CH_3$—N), 3,38-4,00 (breites Multiplett, $CH_2$—N, $CH_2$—O und CH—O).

Elementaranalyse für $C_{45}H_{94}NO_6P$ :

| | | | |
|---|---|---|---|
| Berechnet : | C 69,63 | H 12,21 | N 1,80 |
| Gefunden : | C 69,83 | H 11,85 | N 1,44 (1,40 % Wasser). |

## Beispiel 7

In Analogie zu Beispiel A wurden aus 1 g (2,26 mmol) (RS)-2-O-[(RS)-3,7-Dimethyloctyl]-1-O-[(3RS,7RS)-3,7,11-trimethyldodecyl]glycerin und 1,03 g (3,4 mmol) (4-Hydroxibutyl)-trimethylammonium-p-toluolsulfonat 0,9 g (62,7 %) [4-[[Hydroxi-[(RS)-3-[[(3RS,7RS)-3,7,11-trimethyldodecyl]oxi]-2-[[(RS)-3,7-dimethyloctyl]oxi]propoxi]phosphinyl]oxi]butyl]trimethylammoniumhydroxid (inneres Salz) erhalten.

NMR : 0,80-0,97 (Multiplett, $CH_3$), 1,05-2,15 (breites Multiplett, $CH_2$ und CH), 3,18 (Singlett, $CH_3$—N), 3,38-4,10 (breites Multiplett, $CH_2$—N, $CH_2$—O und CH—O).

Elementaranalyse für $C_{35}H_{74}NO_6P$ :

| | | | |
|---|---|---|---|
| Berechnet : | C 66,10 | H 11,73 | N 2,20 |
| Gefunden : | C 65,61 | H 11,43 | N 2,19 (1,22 % Wasser). |

## Beispiel 8

In Analogie zu Beispiel A wurden aus 1 g (1,92 mmol) (RS)-1-O-[(RS)-3,7-Dimethyloctyl]-2-O-[(3RS,7RS,11RS)-3,7,11,15-tetramethylhexadecyl]glycerin und 1,5 g (4,95 mmol) (4-Hydroxibutyl)-trimethylammonium-p-toluolsulfonat 0,72 g (53,0 %) [4-[[Hydroxi-[(RS)-2-[[(3RS,7RS,11RS)-3,7,11,15-tetramethylhexadecyl]oxi]-3-[[(RS)-3,7-dimethyloctyl]oxi]propoxi]phosphinyl]oxi]butyl]trimethylammoniumhydroxid (inneres Salz) erhalten.

NMR : 0,80-0,95 (Multiplett, $CH_3$), 1,05-2,44 (breites Multiplett, $CH_2$ und CH), 3,30 (breites Singlett, $CH_3$—N), 3,3-4,1 (breites Multiplett, $CH_2$—N, $CH_2$—O und CH—O).

Elementaranalyse für $C_{40}H_{84}NO_6P$ :

| | | | |
|---|---|---|---|
| Berechnet : | C 68,04 | H 11,99 | N 1,98 |
| Gefunden : | C 68,12 | H 12,44 | N 1,95 (1,59 % Wasser). |

Beispiel 9

In Analogie zu Beispiel A wurden aus 1 g (2,26 mmol) (RS)-1-O-[(RS)-Dimethyloctyl]-2-O-[(3RS,7RS)-3,7,11-trimethyldodecyl]glycerin und 1,8 g (5,9 mmol) (4-Hydroxibutyl)-trimethylammonium-p-toluolsulfonat 0,5 g [4-[[Hydroxi-[(RS)-2-[[(3RS,7RS)-3,7,11-trimethyldodecyl]oxi]-3-[[(RS)-3,7-dimethyloctyl]oxi]propoxi]phosphinyl]oxi]butyl] trimethylammoniumhydroxid (inneres Salz) erhalten.

NMR : 0,75-0,95 (Multiplett, $CH_3$), 1,05-2,42 (breites Multiplett, $CH_2$ und CH), 3,34 (breites Singlett, $CH_3$—N), 3,40-4,15 (breites Multiplett, $CH_2$—N, $CH_2$—O und CH—O).

Elementaranalyse für $C_{35}H_{74}NO_6P$ :

Berechnet : C 66,10 H 11,73 N 2,20
Gefunden : C 66,10 H 11,88 N 2,24 (2,73 % Wasser).

Beispiel 10

In Analogie zu Beispiel A wurde aus 0,9 (1,38 mmol) (RS)-2,3-Bis[[(3RS,7R,11R)-3,7,11,15-Tetramethylhexadecyl]oxi]propanol und 0,69 g (2,1 mmol) (4-Hydroxicyclohexyl)-trimethylammonium-p-toluolsulfonat 0,9 g (74,9 %) [(cis/trans)-4-[[[(RS)-2,3-Bis[[(3RS,7R,11R)-3,7,11,15-tetramethylhexadecyl]oxi]propoxi]hydroxiphosphinyl]oxi]cyclohexyl]trimethylammoniumhydroxid (inneres Salz) erhalten.

NMR : 0,80-0,96 (Multiplett, $CH_3$), 1,05-2,44 (breites Multiplett, $CH_2$ und CH), 3,08 (Singlett, $CH_3$—N), 3,33-4,0 (breites Multiplett, CH—N, $CH_2$—O und CH—O).

Elementaranalyse für $C_{52}H_{106}NO_6P$ :

Berechnet : C 71,59 H 12,25 N 1,61
Gefunden : C 71,40 H 12,07 N 1,92 (1,47 % Wasser).

Das (4-Hydroxycyclohexyl)-trimethylammonium-p-toluolsulfonat wurde in Analogie zu Beispiel 1, Abschnitt a) aus cis/trans-4-Dimethylamino-cyclohexanol hergestellt.

Beispiel 11

In Analogie zu Beispiel A wurde aus 0,9 g (2,42 mmol) (RS)-2,3-Bis[[(3RS,7RS)-3,7,11-trimethyldodecyl]oxi]propanol und 0,87 g (2,64 mmol) (4-Hydroxicyclohexyl)-trimethylammonium-p-toluolsulfonat 0,7 g (39,6 %) [(cis/trans)-4-[[[(RS)-2,3-Bis[[(3RS,7RS)-3,7,11-trimethyldodecyl]oxi]propoxi]hydroxiphosphinyl]oxi]cyclohexyl]trimethylammoniumhydroxid (inneres Salz) erhalten.

NMR : 0,80-0,97 (Multiplett, $CH_3$), 1,05-2,75 (breites Multiplett, $CH_2$ und CH), 3,10 (Singlett, $CH_3$—N), 3,35-4,15 (breites Multiplett, CH—N, $CH_2$—O und CH—O).

Elementaranalyse für $C_{42}H_{86}NO_6P$ :

Berechnet : C 68,90 H 11,84 N 1,91
Gefunden : C 68,31 H 11,49 N 1,61 (2,85 % Wasser).

Beispiel 12

In Analogie zu Beispiel A wurden aus 1 g (1,72 mmol) (RS)-1-O-[(3RS,7R,11R)-3,7,11,15-Tetramethylhexyldecyl]-2-O[(3RS,7RS)-3,7,11-trimethyldodecyl]glycerin und 0,86 g (2,6 mmol) (4-Hydroxicyclohexyl)-trimethylammonium-p-toluolsulfonat 0,8 g (58,1 %) [(cis/trans)-4-[[Hydroxi-[(RS)-3-[[(3RS,7R,11R)-3,7,11,15-tetramethylhexydecyl]oxi]-2-[[(3RS,7RS)-3,7,11-trimethyldodecyl]oxi]propoxi]phosphinyl]oxi]-cyclohexyl]trimethylammoniumhydroxid (inneres Salz) erhalten.

NMR : 0,79-0,97 (Multiplett, $CH_3$), 1,00-2,50 (breites Multiplett, $CH_2$ und CH), 3,09 (Singlett, $CH_3$—N), 3,38-3,97 (breites Multiplett, CH—N, $CH_2$—O und CH—O).

Elementaranalyse für $C_{47}H_{96}NO_6P$ :

Berechnet : C 70,37 H 12,06 N 1,75
Gefunden : C 70,00 H 12,13 N 1,99 (1,3 % Wasser).

Beispiel 13

In Analogie zu Beispiel A wurden aus 1,2 g (2,34 mmol) (RS)-2-O-[(RS)-3,7-Dimethyloctyl]-1-O-[(3RS,7R,11R)-3,7,11,15-tetramethylhexadecyl]glycerin und 1,16 g (3,52 mmol) (4-Hydroxicyclohexyl)-trimethylammonium-p-toluolsulfonat 0,96 g (56 %) [(cis/trans)-4-[[Hydroxi-[(RS)-3-[[(3RS,7R,11R)-3,7,11,15-tetramethylhexadecyl]oxi]-2-[[(RS)-3,7-dimethyloctyl]oxi]propoxi]phosphinyl]oxi]cyclohexyl]trimethylammoniumhydroxid (inneres Salz) erhalten.

NMR : 0,80-1,00 (Multiplett, $CH_3$), 1,10-2,50 (breites Multiplett, $CH_2$ und CH), 3,14 (Singlett, $CH_3$—N), 3,40-4,20 (breites Multiplett, CH—N, $CH_2$—O und CH—O).

Beispiel 14

In Analogie zu Beispiel A wurden aus 0,91 (1,56 mmol) (RS)-2-O-[(3RS,7R,11R)-3,7,11,15-Tetramethylhexadecyl]-1-O-[(3RS,7RS)-3,7,11-trimethyldodecyl]glycerin und 0,8 g (2,43 mmol) (4-Hydroxicyclohexyl)-trimethylammonium-p-toluolsulfonat 0,7 g (56 %) [(cis/trans)-4-[[Hydroxi-[(RS)-2-[[(3RS,7R,11R)-3,7,11,15-tetramethylhexadecyl]oxi]-3-[[(3RS,7RS)-3,7,11-trimethyldodecyl]oxi]propoxi]phosphinyl]oxi]cyclohexyl]trimethylammoniumhydroxid (inneres Salz) erhalten.

NMR : 0,78-0,93 (Multiplett, $CH_3$), 1,05-2,44 (breites Multiplett, $CH_2$ und CH), 3,06 (Singlett, $CH_3$—N), 3,37-3,95 (breites Multiplett, CH—N, $CH_2$—O und CH—O).

Elementaranalyse für $C_{47}H_{96}NO_6P$ :

Berechnet : C 70,37 H 12,06 N 1,75
Gefunden : C 70,13 H 12,19 N 1,86 (2,57 % Wasser).

Beispiel 15

In Analogie zu Beispiel A wurden aus 0,9 g (2,03 mmol) (RS)-2-O-[(RS)-3,7-Dimethyloctyl]-1-O-[(3RS,7RS)-3,7,11-trimethyldodecyl]glycerin und 1,12 g (3,4 mmol) (4-Hydroxicyclohexyl)-trimethylammonium-p-toluolsulfonat 1 g (74,3 %) [(cis/trans)-4-[[Hydroxi-[(RS)-3-[[(3RS,7RS)-3,7,11-trimethyldodecyl]oxi]-2-[[(RS)-3,7-dimethyloctyl]oxi]propoxi]phosphinyl]oxi]cyclohexyl]trimethylammoniumhydroxid (inneres Salz) erhalten.

NMR : 0,80-1,00 (Multiplett, $CH_3$), 1,08-2,50 (breites Multiplett, $CH_2$ und CH), 3,14 (Singlett, $CH_3$—N), 3,40-4,00 (breites Multiplett, CH—N, $CH_2$—O und CH—O).

Elementaranalyse für $C_{37}H_{76}NO_6P$ :

Berechnet : C 67,13 H 11,75 N 2,12
Gefunden : C 66,76 H 11,35 N 2,05 (1,06 % Wasser).

Beispiel 16

In Analogie zu Beispiel A wurden aus 0,9 g (1,76 mmol) (RS)-1-O-[(RS)-3,7-Dimethyloctyl]-2-O-[(3RS,7RS,11RS)-3,7,11,15-trimethylhexadecyl]glycerin und 1,2 g (3,64 mmol) (4-Hydroxicyclohexyl)-trimethylammonium-p-toluolsulfonat 0,4 g [(cis/trans)-4-[[Hydroxi-[(RS)-2-[[(3RS,7RS,11RS)-3,7,11,15-tetramethylhexadecyl]oxi]-3-[[(RS)-3,7-dimethyloctyl]oxi]propoxi]phosphinyl]oxi]cyclohexyl]trimethylammoniumhydroxid (inneres Salz) erhalten.

NMR : 0,77-0,95 (Multiplett, $CH_3$), 1,00-2,65 (breites Multiplett, $CH_2$ und CH), 3,20 (Singlett, $CH_3$—N), 3,33-4,23 (breites Multiplett, CH—N, $CH_2$—O und CH—O).

Elementaranalyse für $C_{42}H_{86}NO_6P$ :

Berechnet : C 68,90 H 11,84 N 1,91
Gefunden : C 68,62 H 11,55 N 1,74 (2,83 % Wasser).

Beispiel 17

In Analogie zu Beispiel A wurden aus 1 g (2,26 mmol) (RS)-1-O-[(RS)-Dimethyloctyl]-2-O-[(3RS,7RS)-3,7,11-trimethyldodecyl]glycerin und 2,1 g (6,37 mmol) (4-Hydroxicyclohexyl)-trimethylammonium-p-toluolsulfonat 0,4 g [(cis/trans)-4-[[Hydroxi-[(RS)-2-[[(3RS,7RS)-3,7,11-trimethyldodecyl]oxi]-3-[[(RS)-3,7-dimethyloctyl]oxi]propoxi]phosphinyl]oxi]cyclohexyl]trimethylammoniumhydroxid (inneres Salz) erhalten.

NMR : 0,77-0,85 (Multiplett, $CH_3$), 1,00-1,83 und 2,08-2,58 (2 breite Multipletts, $CH_2$ und CH), 3,25 (breites Singlett, $CH_3$—N), 3,33-4,25 (breites Multiplett, CH—N, $CH_2$—O und CH—O).

Beispiel 18

In Analogie zu Beispiel A wurden aus 1 g (1,53 mmol) (RS)-2,3-Bis[[(3RS,7R,11R)-3,7,11,15-tetramethylhexadecyl]oxi]propanol und 1,3 g (4,3 mmol) 4-Hydroxi-1,1-dimethylpiperidinium-p-toluolsulfonat 0,5 g (38,7 %) 4-[[[(RS)-2,3-Bis[[(3RS,7RS,11RS)-3,7,11,15-Tetramethylhexadecyl]oxi]propoxi]hydroxiphosphinyl]oxi]-1,1-dimethylpiperidiniumhydroxid (inneres Salz) erhalten.

NMR : 0,78-0,93 (Multiplett, $CH_3$), 0,97-1,72 (breites Multiplett, $CH_2$ und CH, Alkylkette), 2,00-2,30 und 2,70-2,97 (2 breite Multipletts, $CH_2$, Ring), 3,29 (breites Singlett, $CH_3$—N), 3,34-2,98 (breites Multiplett, $CH_2$—N, $CH_2$—O und CH—O—P), ca. 4,44 (breites Multiplett, CH—O).

Elementaranalyse für $C_{50}H_{102}NO_6P$ :

Berechnet : C 71,13 H 12,18 N 1,66
Gefunden : C 70,42 H 12,03 N 1,68 (0,57 % Wasser).

Das 4-Hydroxy-1,1-dimethylpiperidinium-p-toluolsulfonat wurde in Analogie zu Beispiel A, Abschnitt a) aus 4-Hydroxy-1-methylpiperidin hergestellt.

Beispiel 19

In Analogie zu Beispiel A erhält man aus (RS)-2,3-Bis[[(3RS, 7RS, 11RS)-3,7,11,15-tetramethylhexadecyl]oxi]-propanol und 1-[2-Hydroxiäthyl]-1-methyl-piperidinium-p-toluolsulfonat 1-[2-[[[(RS)-2,3-Bis[[(3RS, 7RS, 11RS)-3,7,11,15-tetramethylhexadecyl]oxi]propoxi]hydroxiphosphinyl]oxi]äthyl]-1-methylpiperidiniumhydroxid (inneres Salz). Ausbeute 76 %

NMR : 0,86-0,91 (Multiplett, 10 $CH_3$), 1,05-1,70 (breites Multiplett, $CH_2$ und CH), 1,89-2,15 (Multiplett, 3 $CH_2$ im Piperidinring); 3,18 (Singlett, $CH_3—N$), 3,36-3,74, 3,88 und 4,23-4,32 (breites Multiplett, Triplett und Multiplett, $CH_2—O$, CHO und $CH_2—N$).

Das 1-[2-Hydroxiäthyl]-1-methyl-piperidinium-p-toluolsulfonat kann in Analogie zu Beispiel Aa) aus 1-(2-Hydroxiäthyl)-piperidin erhalten werden.

Beispiel 20

1,0 g der in Beispiel 2 hergestellten Verbindung, 2,4 g Saccharose und 7,5 ml Wasser werden mit einem Magnetrührer 1 Stunde lang intensiv gerührt. Dabei entsteht eine milchige Dispersion, die hauptsächlich multilamellare Liposomen enthält.

Diese Dispersion wird anschliessend (bei 20 °C, pH 7,0 und unter $N_2$-Begasung) 20 Minuten lang mit Ultraschall behandelt (Branson Sonifier B-12), wobei sich eine schwach opaleszierende Liposomen-Lösung bildet, die zum grössten Teil aus kleinen, monolamellaren Liposomen besteht.

Zur Entfernung von gröberen Partikeln zentrifugiert man die Lösung anschliessend und filtriert sie durch 0,22 μ-Millipore-Filter. Dann wird sie in Ampullen abgefüllt und Hitze-sterilisiert (20 Minuten bei 120 °C).

Beispiel 21

In der in Beispiel 20 erhaltenen schwach opaleszierenden Liposomenlösung werden vor der Filtration 4,1 mg/ml Diazepam durch Einrühren aufgelöst. Anschliessend verfährt man wie oben. Man erhält dabei eine Diazepam-Injektionslösung, die über Monate stabil ist.

Beispiel 22

0,78 g Na-Glycocholat und 1,02 g der in Beispiel 2 hergestellten Verbindung werden in 10 ml Methanol aufgelöst. Durch rasches Verdampfen am Vakuum erzeugt man aus dieser Lösung an der Wand eines Kolbens einen dünnen Film. Dieser wird durch die Zugabe von 8,38 ml Wasser wieder aufgelöst, wobei eine klare Mischmicellen-Lösung entsteht.

Nach der Filtration durch 0,22 μ-Millipore-Filter wird die Micell-Lösung in Ampullen abgefüllt und sterilisiert (20 Minuten bei 120 °C).

**Patentansprüche** (für die Vertragsstaaten : BE, CH, DE, FR, GB, IT, LI, LU, NL, SE)

1. Verbindungen der allgemeinen Formel

$$\begin{array}{l} CH_2-O-R^1 \\ | \\ CH\ -O-R^2 \\ | \\ CH_2-O-R^3 \end{array} \qquad (I)$$

worin zwei der Reste $R^1$, $R^2$ und $R^3$ $C_{10-30}$-Alkylreste mit mindestens 8 C-Atomen in gerader Kette darstellen, wobei mindestens einer dieser Reste durch mindestens 2 $C_{1-3}$-Alkylreste substituiert ist und die Summe der C-Atome in beiden Resten grösser als 20 ist ; und der dritte Rest ein Rest $—P(O)(O^-)OR^4$ ist, worin $R^4$ einen durch eine quaternäre Ammoniumgruppe substituierten Butyl- oder $C_{5-7}$-Cycloalkylrest oder einen ein di-($C_{1-6}$-Alkyl)-substituiertes Stickstoffatom enthaltenden $C_{5-7}$-Cycloalkylrest darstellt.

2. Verbindungen gemäss Anspruch 1, wobei $R^1$ und $R^2$ $C_{10-30}$-Alkylreste mit mindestens 8 C-Atomen in gerader Kette darstellen, die jeweils durch mindestens 2 $C_{1-3}$-Alkylreste substituiert sind.

3. Verbindungen gemäss Anspruch 2, in denen zwei der Reste $R^1$, $R^2$ und $R^3$ Hexahydrofarnesyl oder Dihydrophytyl darstellen.

4. Verbindungen gemäss den Ansprüchen 1-3, in denen $R^4$ Tri-($C_{1-6}$-alkyl)-ammonio-$C_{5-7}$-cycloalkyl oder N,N-Di-$C_{1-6}$-alkyl-$C_{4-6}$-azacycloalkyl darstellt.

5. Verbindungen gemäss den Ansprüchen 1-4, in denen $R^4$ 4-(Trimethylammonio)butyl, 4-(Trimethylammonio)cyclohexyl oder N,N-Dimethyl-4-piperidyl ist.

6. [4-[[[(RS)-2,3-Bis[[(3RS, 7RS, 11RS)-3,7,11,15-Tetramethylhexadecyl]oxi]propoxi]hydroxiphosphinyl]oxi]-butyl]trimethyl-ammoniumhydroxid (inneres Salz).

7. Verbindungen gemäss den Ansprüchen 1-6 zur Anwendung bei der Herstellung kolloidaler Lösungsmittelsysteme.

8. Liposomen- und Mischmicell-Lösungen auf der Basis einer Verbindung der Formel I.

9. Verwendung von Verbindungen der Formel I zur Herstellung kolloidaler Lösungsmittelsysteme.

**Patentansprüche** (für den Vertragsstaat AT)

1. Verfahren zur Herstellung von Verbindungen der allgemeinen Formel

$$\begin{array}{l} CH_2\text{-}O\text{-}R^1 \\ | \\ CH\ \text{-}O\text{-}R^2 \\ | \\ CH_2\text{-}O\text{-}R^3 \end{array} \qquad (I)$$

worin zwei der Reste $R^1$, $R^2$ und $R^3$ $C_{10-30}$-Alkylreste mit mindestens 8 C-Atomen in gerader Kette darstellen, wobei mindestens einer dieser Reste durch mindestens 2 $C_{1-3}$-Alkylreste substituiert ist und die Summe der C-Atome in beiden Resten grösser als 20 ist ; und der dritte Rest ein Rest —P(O)(O⁻)OR$^4$ ist, worin $R^4$ einen durch eine quaternäre Ammoniumgruppe substituierten Butyl- $C_{5-7}$-Cycloalkylrest oder einen ein di-($C_{1-6}$-Alkyl)-substituiertes Stickstoffatom enthaltenden $C_{5-7}$-Cycloalkylrest darstellt dadurch gekennzeichnet, dass man einen Glycerinäther der allgemeinen Formel

$$\begin{array}{l} CH_2\text{-}O\text{-}R^{11} \\ | \\ CH\ \text{-}O\text{-}R^{21} \\ | \\ CH_2\text{-}O\text{-}R^{31} \end{array} \qquad (II)$$

wobei einer der Reste $R^{11}$, $R^{21}$ und $R^{31}$ Wasserstoff und die verbleibenden Reste einen wie in Anspruch 1 definierten $C_{10-30}$-Alkylrest darstellen,

a) mit Phosphoroxychlorid in Gegenwart einer Base und danach mit einem Alkohol $R^4OH$, worin $R^4$ die oben angegebene Bedeutung hat, umsetzt, oder

b) in Gegenwart einer Base mit einer Verbindung der Formel

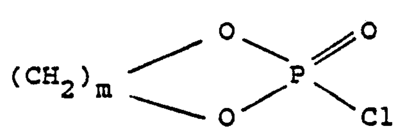

(III)

worin m 2 oder 3 ist, und danach mit einem Tri-$C_{1-6}$-alkylamin umsetzt.

2. Verfahren nach Anspruch 1, dadurch gekennzeichnet, dass man Verbindungen der Formel I herstellt, in denen $R^1$ und $R^2$ $C_{10-30}$-Alkylreste mit mindestens 8 C-Atomen in gerader Kette darstellen, die jeweils durch mindestens 2 $C_{1-3}$-Alkylreste substituiert sind.

3. Verfahren nach Anspruch 2, dadurch gekennzeichnet, dass man Verbindungen der Formel I herstellt, in denen zwei der Reste $R^1$, $R^2$ und $R^3$ Hexahydrofarnesyl oder Dihydrophytyl darstellt.

4. Verfahren nach den Ansprüchen 1-3, dadurch gekennzeichnet, dass man Verbindungen der Formel I herstellt, in denen $R^4$ Tri-($C_{1-6}$-alkyl)-ammonio-$C_{5-7}$-cycloalkyl oder N,N-Di-$C_{1-6}$-alkyl-$C_{4-6}$-azacycloalkyl darstellt.

5. Verfahren nach den Ansprüchen 1-4, dadurch gekennzeichnet, dass man Verbindungen der Formel I herstellt, in denen $R^4$ 4-(Trimethylammonio)butyl, 4-(Trimethylammonio)cyclohexyl oder N,N-Dimethyl-4-piperidyl ist.

6. Verfahren nach Anspruch 1, dadurch gekennzeichnet, dass man [4-[[[(RS)-2,3-Bis[[(3RS, 7RS, 11RS)-3,7,11,15-Tetramethylhexadecyl]oxi]propoxi]hydroxiphosphinyl]oxi]-butyl]trimethyl-ammoniumhydroxid (inneres Salz) herstellt.

**Claims** (for the Contracting States : BE, CH, DE, FR, GB, IT, LI, LU, NL, SE)

1. Compounds of the general formula

$$\begin{array}{l} CH_2-O-R^1 \\ | \\ CH\ -O-R^2 \\ | \\ CH_2-O-R^3 \end{array} \qquad (I)$$

wherein two of the residues $R^1$, $R^2$ and $R^3$ represent $C_{10-30}$-alkyl residues with at least 8 C-atoms in a straight-chain, at least one of the residues being substituted by at least 2 $C_{1-3}$-alkyl residues and the sum of the C-atoms in the two residues being greater than 20 ; and the third residue is a residue $-P(O)(O^-)OR^4$ in which $R^4$ represents a butyl or $C_{5-7}$-cycloalkyl residue which is substituted by a quaternary ammonium group or a $C_{5-7}$-cycloalkyl residue which contains a di-($C_{1-6}$-alkyl)-substituted nitrogen atom.

2. Compounds in accordance with claim 1, in which $R^1$ and $R^2$ represent $C_{10-30}$-alkyl residues with at least 8 C-atoms in a straight-chain, each of which is substituted by at least 2 $C_{1-3}$-alkyl residues.

3. Compounds in accordance with claim 2, in which two of the residues $R^1$, $R^2$ and $R^3$ represent hexahydrofarnesyl or dihydrophytyl.

4. Compounds in accordance with claims 1-3, in which $R^4$ represents tri-($C_{1-6}$-alkyl)-ammonio-$C_{5-7}$-cycloalkyl or N,N-di-$C_{1-6}$-alkyl-$C_{4-6}$-azacycloalkyl.

5. Compounds in accordance with claims 1-4, in which $R^4$ is 4-(trimethylammonio)butyl, 4-(trimethylammonio)-cyclohexyl or N,N-dimethyl-4-piperidyl.

6. [4-[[[(RS)-2,3-Bis[[(3RS, 7RS, 11RS)-3,7,11,15-tetramethylhexadecyl]oxy]propoxy]hydroxyphosphinyl]oxy]butyl]-trimethyl-ammonium hydroxide (internal salt).

7. Compounds in accordance with claims 1-6 for use in the manufacture of colloidal solvent systems.

8. Liposome and mixed micelle solutions based on a compound of formula I.

9. The use of compounds of formula I for the manufacture of colloidal solvent systems.

**Claims** (for the Contracting State AT)

1. A process for the manufacture of compounds of the general formula

$$\begin{array}{l} CH_2-O-R^1 \\ | \\ CH\ -O-R^2 \\ | \\ CH_2-O-R^3 \end{array} \qquad (I)$$

wherein two of the residues $R^1$, $R^2$ and $R^3$ represent $C_{10-30}$-alkyl residues with at least 8 C-atoms in a straight-chain, at least one of the residues being substituted by at least 2 $C_{1-3}$-alkyl residues and the sum of the C-atoms in the two residues being greater than 20 ; and the third residue is a residue $-P(O)(O^-)OR^4$ in which $R^4$ represents a butyl or $C_{5-7}$-cycloalkyl residue which is substituted by a quaternary ammonium group or a $C_{5-7}$-cycloalkyl residue which contains a di-($C_{1-6}$-alkyl)-substituted nitrogen atom.

characterized by reacting a glycerol ether of the general formula

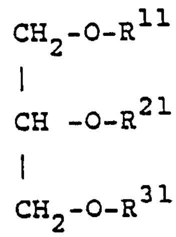

(II)

wherein one of the residues $R^{11}$, $R^{21}$ and $R^{31}$ represents hydrogen and the remaining residues represent a $C_{10-30}$-alkyl residue as defined in claim 1

a) with phosphorus oxychloride in the presence of a base and thereafter with an alcohol $R^4OH$, wherein $R^4$ has the significance given above, or

b) in the presence of a base with a compound of the formula

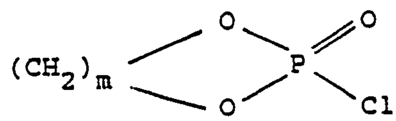

(III)

wherein m is 2 or 3, and thereafter with a tri-$C_{1-6}$-alkylamine.

2. A process according to claim 1, characterized in that compounds of formula I in which $R^1$ and $R^2$ represent $C_{10-30}$-alkyl residues with at least 8 C-atoms in a straight-chain, each of which is substituted by at least 2 $C_{1-3}$-alkyl residues, are manufactured.

3. A process according to claim 2, characterized in that compounds of formula I in which two of the residues $R^1$, $R^2$ and $R^3$ represent hexahydrofarnesyl or dihydrophytyl are manufactured.

4. A process according to claims 1-3, characterized in that compounds of formula I in which $R^4$ represents tri-($C_{1-6}$-alkyl)-ammonio-$C_{5-7}$-cycloalkyl or N,N-di-$C_{1-6}$-alkyl-$C_{4-6}$-azacycloalkyl are manufactured.

5. A process according to claims 1-4, characterized in that compounds of formula I in which $R^4$ is 4-(trimethylammonio)butyl, 4-(trimethylammonio)cyclohexyl or N,N-dimethyl-4-piperidyl are manufactured.

6. A process according to claim 1, characterized in that [4-[[[(RS)-2,3-bis[[(3RS, 7RS, 11RS)-3,7,11,15-tetramethylhexadecyl]oxy]propoxy]hydroxyphosphinyl]oxy]butyl]-trimethyl-ammonium hydroxide (internal salt) is manufactured.

**Revendications** (pour les Etats contractants : BE, CH, DE, FR, GB, IT, LI, LU, NL, SE)

1. Composés de formule générale :

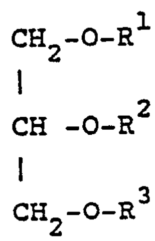

(I)

dans laquelle deux des symboles $R^1$, $R^2$ et $R^3$ représentent des groupes alkyle en $C_{10}$-$C_{30}$ avec au moins 8 atomes de carbone en chaîne droite, l'un au moins de ces groupes étant substitué par au moins deux groupes alkyle en $C_1$-$C_3$ et la somme des atomes de carbone dans les deux groupes étant supérieure à 20 ; et le troisième groupe est un groupe $—P(O)(O^-)OR^4$ dans lequel $R^4$ représente un groupe butyle ou cycloalkyle en $C_5$-$C_7$ substitué par un groupe ammonium quaternaire ou un groupe cycloalkyle en $C_5$-$C_7$ contenant un atome d'azote substitué par deux groupes alkyle en $C_1$-$C_6$.

2. Composés selon la revendication 1, dans lesquels $R^1$ et $R^2$ représentent des groupes alkyle en $C_{10}$-$C_{30}$ avec au moins 8 atomes de carbone en chaîne droite, et qui sont chacun substitués par au moins deux groupes alkyle en $C_1$-$C_3$.

3. Composés selon la revendication 2, dans lesquels deux des symboles $R^1$, $R^2$ et $R^3$ représentent des groupes hexahydrofarnésyle ou dihydrophytyle.

4. Composés selon les revendications 1 à 3, dans lesquels $R^4$ représente un groupe tri-(alkyle en $C_1$-$C_6$)-ammoniocycloalkyle en $C_5$-$C_7$ ou N,N-di-(alkyle en $C_1$-$C_6$)-azacycloalkyle en $C_4$-$C_6$.

5. Composés selon les revendications 1 à 4, dans lesquels $R^4$ représente un groupe 4-(triméthylammonio)-butyle, 4-(triméthylammonio)-cyclohexyle ou N,N-diméthyl-4-pipéridyle.

6. L'hydroxyde de [4-[[[(RS)-2,3-bis-[[(3RS, 7RS, 11RS)-3,7,11,15-tétraméthylhexadécyl]-oxy]-propoxy]-hydroxyphosphinyl]-oxo]-butyl]-triméthylammonium (sel interne).

7. Composés selon les revendications 1 à 6, pour l'utilisation dans la préparation de système solvants colloïdaux.

8. Solutions à liposomes et à micelles mélangées à base d'un composé de formule I.

9. Utilisation de composés de formule I pour la préparation de systèmes solvants colloïdaux.

**Revendications** (pour l'Etat contractant AT)

1. Procédé de préparation des composés de formule générale :

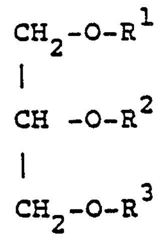

(I)

dans laquelle deux des symboles $R^1$, $R^2$ et $R^3$ représentent des groupes alkyle en $C_{10}$-$C_{30}$ à au moins 8 atomes de carbone en chaîne droite, l'un au moins de ces groupes étant substitué par au moins deux groupes alkyle en $C_1$-$C_3$ et la somme des atomes de carbone des deux groupes étant supérieure à 20 ; et le troisième groupe est un groupe $P(O)(O^-)OR^4$ dans lequel $R^4$ représente un groupe butyle ou cycloalkyle en $C_5$-$C_7$ substitué par un groupe ammonium quaternaire ou un groupe cycloalkyle en $C_5$-$C_7$ contenant un atome d'azote substitué par deux groupes alkyle en $C_1$-$C_6$, caractérisé en ce que l'on fait réagir un éther du glycérol de formule générale :

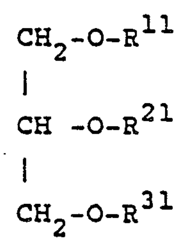

(II)

dans laquelle l'un des symboles $R^{11}$, $R^{21}$ et $R^{31}$ représente l'hydrogène et les autres représentent chacun un groupe alkyle en $C_{10}$-$C_{30}$ tel que défini dans la revendication 1,

(a) avec l'oxychlorure de phosphore en présence d'une base puis avec un alcool $R^4OH$ dans lequel $R^4$ a les significations indiquées ci-dessus, ou bien

(b) en présence d'une base avec un composé de formule

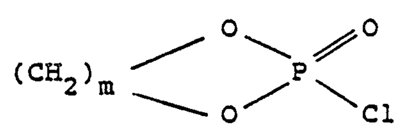

(III)

dans laquelle m est égal à 2 ou 3, puis avec une tri-(alkyle en $C_1$-$C_6$)-amine.

2. Procédé selon la revendication 1, caractérisé en ce que l'on prépare des composés dans lesquels $R^1$ et $R^2$ représentent des groupes alkyle en $C_{10}$-$C_{30}$ avec au moins 8 atomes de carbone en chaîne droite, qui sont substitués chacun par au moins deux groupes alkyle en $C_1$-$C_3$.

3. Procédé selon la revendication 2, caractérisé en ce que l'on prépare des composés de formule I dans lesquels deux des symboles $R^1$, $R^2$ et $R^3$ représentent des groupes hexahydrofarnésyle ou dihydrophytyle.

4. Procédé selon les revendications 1 à 3, caractérisé en ce que l'on prépare des composés de formule I dans lesquels $R^4$ représente un groupe tri-(alkyle en $C_1$-$C_6$)-ammoniocycloalkyle en $C_5$-$C_7$ ou N,N-di-(alkyle en $C_1$-$C_6$)-azacycloalkyle en $C_4$-$C_6$.

5. Procédé selon les revendications 1 à 4, caractérisé en ce que l'on prépare des composés de formule I dans lesquels $R^4$ représente un groupe 4-(triméthylammonio)-butyle, 4-(triméthylammonio)-cyclohexyle ou N,N-diméthyl-4-pipéridyle.

6. Procédé selon la revendication 1, caractérisé en ce que l'on prépare l'hydroxyde de [4-[[[(RS)-2,3-bis-[[(3RS, 7RS, 11RS)-3,7,11,15-tétraméthylhexadécyl]-oxy]-propoxy]-hydroxyphosphinyl]-oxo]-butyl]-triméthylammonium (sel interne).